# EUROPEAN PATENT SPECIFICATION

(11) **EP 0 488 850 B1**
(45) Date of publication and mention of the grant of the patent: **23.08.1995**
(21) Application number: 91403092.9
(22) Date of filing: 18.11.1991
(51) Int. Cl.: A47C 27/14, A47C 27/00, A61N 2/06

(54) **Mattress for magnetic treatment**
Matratze zur magnetischen Behandlung
Matelas pour traitement magnétique

(30) Priority: 27.11.1990 JP 323808/90
(43) Date of publication of application: 03.06.1992
(73) Proprietor: JAPAN LIFE COMPANY LIMITED, Toshima-ku Tokyo (JP)
(72) Inventor: Yamaguchi, Takayoshi, c/o Japan Life., Ltd., Toshima-ku, Tokyo (JP); Kobayashi, Isamu, c/o Japan Life., Ltd., Toshima-ku, Tokyo (JP)
(74) Representative: Keib, Gérard

(56) References cited:
- EP-A- 0 137 072
- US-A- 4 330 892
- US-A- 4 924 542
- US-A- 5 035 017

## Description

### BACKGROUND OF THE INVENTION

### Field of the Invention:

The present invention relates to a mattress for magnetic treatment which produces magnetic treatment effects by permanent magnets and chiropractic effects by protuberances while sleeping.

### Description of the Prior Art:

Magnetism has been known to produce various magnetic treatment effects as it promotes circulation of the blood and relieves fatigue by freeing stiffness in the muscles. Varieties of products incorporating permanent magnets have also been sold on the market and a magnetic mattress is typical of many.

However, most of the conventional magnetic mattresses are only dotted with single permanent magnets on their surfaces (e.g., Japanese Utility Model Publication No. 6148/1989).

The conventional magnetic mattresses are often dotted with permanent magnets on their surfaces as stated above and because the magnets are widely spaced apart, the magnetic flux tends to concentrate on the vicinity of the surface of a magnetic material forming the permanent magnet. As a result, a high-density magnetic field is hardly obtainable at a place far from the magnet and the magnet effect is barely produced in a deep part of the human body on the mattress. In other words, satisfactory magnetic treatment effects are not anticipated.

In US-A-4,330,892 is described a mattress comprising a principal constituent on which is placed a first felt mat and a second felt mat placed on said first felt mat. The second felt mat comprises a number of holes which contain permanent magnet pieces.

In EP-A-0 137 072 is described a sleeping mattress which comprises a structure of four layers in which a first layer is a wave layer, a second layer is a magnetic layer including magnets, a third layer is a compression palm lock layer, and a fourth layer is a cushion layer.

### SUMMARY OF THE INVENTION

An object of the present invention is to provide a mattress for magnetic treatment which generates magnetic lines of force that draw a high-density loop and effectively act on even a deep part of the human body, and thereby produces satisfactory magnetic treatment effects.

Another object of the present invention is to provide a mattress for magnetic treatment which produces appropriate chiropractic effects as well as magnetic treatment effects.

A mattress for magnetic treatment according to the present invention comprises an elastic body of crosslinked foamed polyethylene, foamed urethane or the like, the elastic body having protuberances on its surface for producing chiropractic effects, damper felt fixed to the surface of the elastic body, the damper felt being dotted with permanent magnets, a fiber layer fixed to the surface of the damper felt and used for holding the permanent magnets and the protuberances of the elastic body, and a thin soft fiber layer covering the surface of the fiber layer, characterized in that said elastic body (1) includes short (1a) and tall (1b) barrel-like bodies joined and arranged in rows and columns.

With this arrangement, magnetism is allowed to infiltrate into a deep part of the human body so as to produce satisfactory magnetic treatment effects as the permanent magnets thus disposed have high-density magnetism flying intensely over the surface in contact with the human body.

Moreover, the protuberances provided on the elastic body produce appropriate chiropractic effects.

Dependent claims 2 to 5 define various modification of the mattress of claim 1.

Other and further objects, features and advantages of the present invention will appear more fully from the following description.

### BRIEF DESCRIPTION OF THE DRAWINGS

Fig. 1 is a partial cutaway perspective view of an embodiment of the present invention.

Fig. 2 is a partial enlarged top view of Fig. 1.

Fig. 3 is a sectional view taken on line A - A of Fig. 2.

Fig. 4 is a sectional view taken on line B - B of Fig. 2.

Fig. 5 is a vertical sectional view of another embodiment of the present invention.

Fig. 6 is a partial cutaway perspective view of still another embodiment of the present invention.

Fig. 7 is a partial enlarged top view of Fig. 6.

Fig. 8 is a sectional view taken on line C - C of Fig. 7.

### DESCRIPTION OF THE PREFERRED EMBODIMENTS

Referring to the accompanying drawings, preferred embodiments of the present invention will be described.

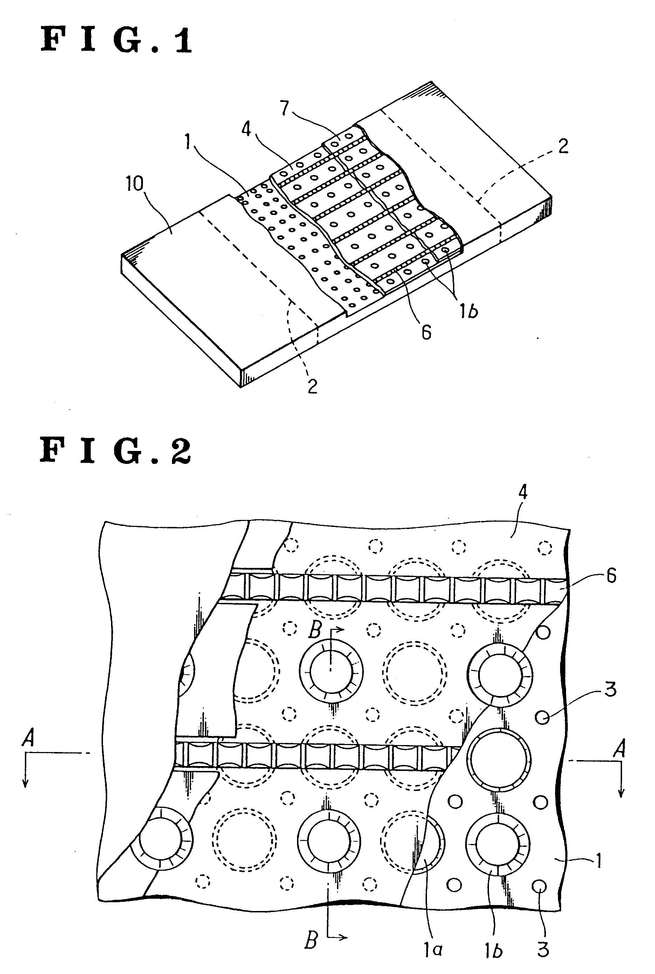

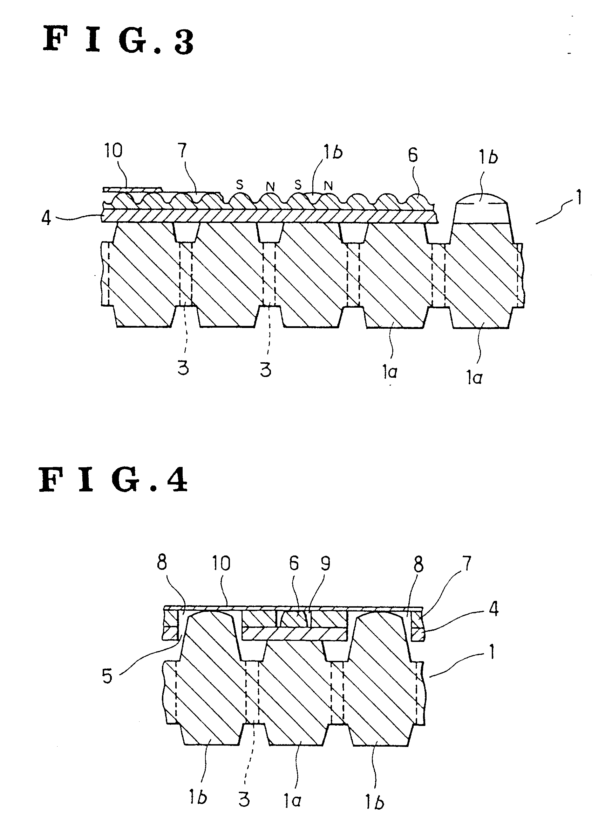

First, an embodiment of the present invention shown in Figs. 1 to 4 inclusive, will be described. Fig. 1 is a partial cutaway perspective view of the embodiment shown, wherein a turnup 2 is formed at two places (when folded in three) to make foldable a relatively thin elastic body 1 of crosslinked foamed polyethylene, foamed urethane or the like. The elastic body 1 is in such a form that a number of barrel-like bodies are coupled together crosswise, with an air vent 3 which is a vertical through-hole and provided in each barrel-to-barrel coupling portion. The air vents 3 are intended to smooth the circulation of air through the whole mattress. Among the barrel-like bodies, there are a large number of short barrel-like bodies 1a and a small number of tall barrel-like bodies 1b, these being disposed at fixed intervals. Damper felt 4 made of mixed-spun felt is adhesion-bonded to the surface of the elastic body 1. A through-hole 5 is bored in a portion of the damper felt 4 corresponding in position to the tall barrel-like body 1b and the head of the tall barrel-like body 1b is exposed therethrough (see Fig. 4 especially). Several rows of continuous magnetizing magnets 6 extending in the longitudinal direction of the mattress are secured to the surface of the damper felt 4 with an adhesive, for instance. The continuous magnetizing magnet 6 is formed by alternately N- and S-poles on a magnetic material mainly composed of rubber, plastics or the like and having parallel conical heads semicircular in section as shown in Fig. 3. The magnetic line of force is generated from the continuous magnetizing magnet 6 in such a way that it is directed from the crest of the N-seeking conical head to that of the adjoining S-pole conical head. As each conical head is isolated from the adjoining one with a valley there between, the magnetic path becomes long and the magnetic line of force is generated vertically. In other words, the magnetic flux density is rendered higher at a position away from both magnetic poles. Therefore, the magnetic lines of force reach a deep part of the human body lying down on the mattress having the magnets for magnetic treatment as described above and satisfactory magnetic treatment effects are produced. Moreover, the conical heads produce substitute chiropractic effects.

The tall barrel-like body 1b is disposed between the parallel continuous magnetizing magnets 6. The damper felt 4 is located beneath the continuous magnetizing magnets 6 and prevents these magnets from sinking.

A soft fiber layer 7 formed of synthetic fiber cotton is secured to the surface of the damper felt 4 by knitting or bonding. The fiber layer 7 is arranged so that it is about the same height as that of the continuous magnetizing magnets 6 on the damper felt 4. Moreover, the fiber layer 7 is provided with through-holes 8 for relieving the heads of the respective rall barrel-like bodies 1b, each hole communicating with the through-hole 5 of the damper felt. The fiber layer 7 is also provided with longitudinal grooves 9 for relieving the respective continuous magnetizing magnets 6. Further, a thin soft fiber layer 10 made of unwoven fabric is affixed to the surface of the fiber layer 7 by knitting.

When the mattress thus completed according to the present invention is used, a sheet may be laid out thereon or a bag-like cover may be employed for wrapping the mattress.

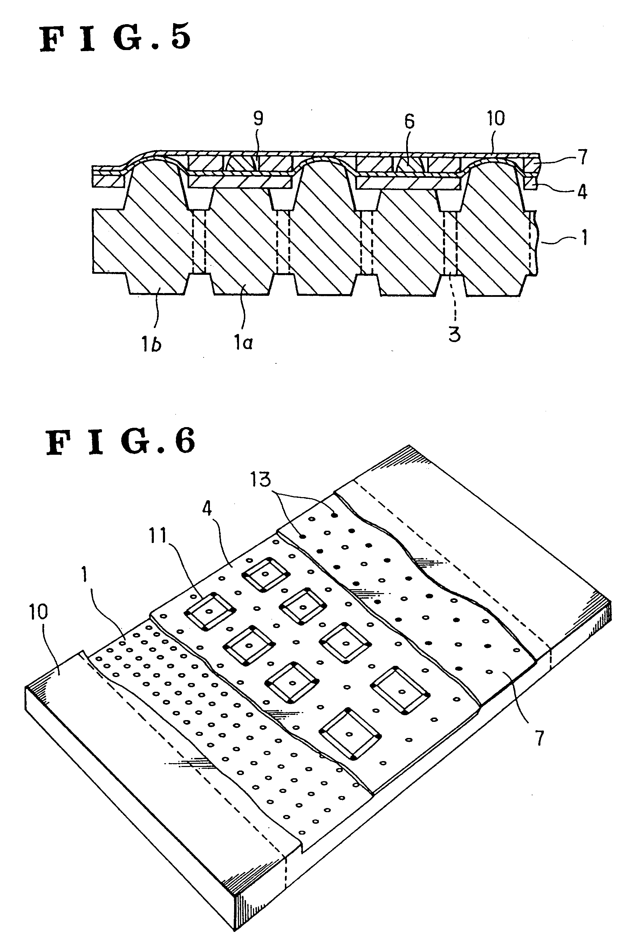

In another embodiment of Fig. 5, the thin layer 10 in the previous embodiment is formed into a bag for enclosing the continuous magnetizing mangets 6 and the fiber layer 7 and this bag is affixed to the damper felt 4 by bonding or knitting.

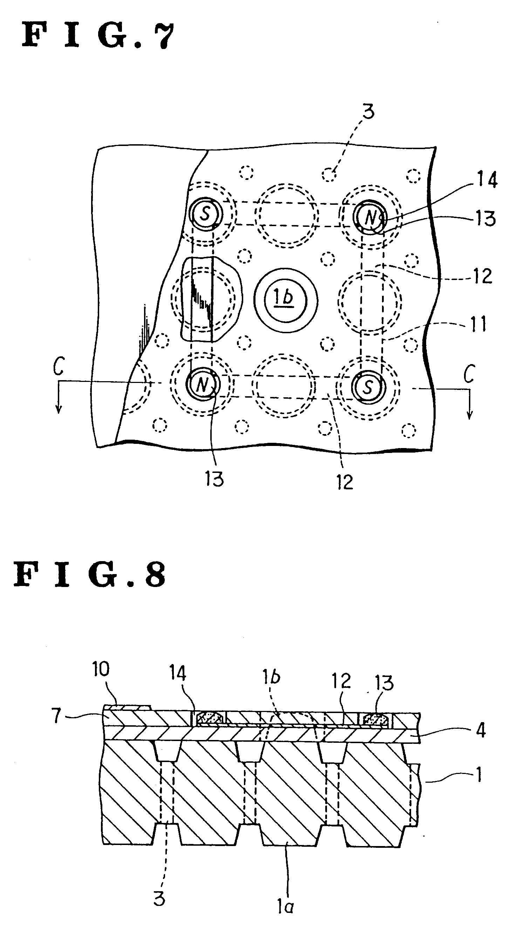

Figs. 6 to 8 inclusive, refers to an embodiment wherein square multipolar magnets 11 in place of the continuous magnetizing magnets 6 in the previous embodiment are employed. The multipolar magnet 11 comprises magnetic binding plates 12 disposed in the form of a square and magnetic materials 13 secured to the respective corners of the square, the magnetic materials 13 adjacent to each other being oppositely polarized. The combination of the binding plates 12 may be what has been preformed integrally into such a square. The multipolar magnet 11 is square in shape and one side thereof forms a double-pole magnet. As the magnetic lines of force generated from the combination of magnetic poles are caused to fly high in a concentrated manner, waste of time is eliminated. The magnetic line of force generated from the N-seeking magnetic pole flies to return to the S-seeking magnetic pole and proceeds from the S-seeking magnetic pole to the N-seeking magnetic pole through the binding plate 12. As the magnetic poles are sufficiently spaced apart, the magnetic lines of force strongly act on the human body and promote the circulation of the blood. The magnetic treatment effect is thus promoted.

Even in this embodiment, the damper felt 4 is fixedly secured to the surface of the elastic body 1 and the fiber layer 7 is further secured to the surface of that combination. A through-hole 14 for use in relieving each magnetic material 12 of the multipolar magnet 11 fixed onto the damper felt 4 is formed in the fiber layer 7 . Moreover, there is provided a shallow recess for accommodating the binding plate 12 as occasion demands. The remaining part of the construction is similar to what has been described above.

As set forth above, the provision of the continuous magnetizing magnets or multipolar magnets allows high-density magnetism to infiltrate into a deep part of the human body with the effect of promoting the circulation of the blood in various parts thereof so as to free the stiffness in the muscles. In addition to the excellent magnetic treatment effects, the elastic body equipped with the protuberances produce chiropractic effects appropriate to the human body, so that the mattress according to the present invention contributes to not only fatigue recovery but also maintenance of health with the treatment effects described above.

As many apparently widely different embodiments of this invention may be made without departing from the scope thereof, it is to be understood that the invention is not limited to the specific embodiments thereof except as defined in the appended claims.

## Claims

1. A mattress for magnetic treatment comprising an elastic body (1) of crosslinked foamed polyethylene or foamed urethane, the elastic body (1) having protuberances on its surface for producing chiropractic effects; a damper felt (4) fixed to the surface of the elastic body (1), the damper felt (4) being dotted with permanent magnets (6); a fiber layer (7) fixedly secured to the surface of the damper felt (4) and used for holding the permanent magnets (6) and the protuberances of the elastic body (1); a thin soft fiber layer (10) covering the surface of the fiber layer (7); said elastic body (1) including short (1a) and tall (1b) barrel-like bodies joined and arranged in rows and columns.

2. A mattress for magnetic treatment as claimed in claim 1, wherein a vertical through-hole (5) is provided in each barrel-to-barrel coupling portion.

3. A mattress for magnetic treatment as claimed in claim 1, wherein said permanent magnets take the poles form of a magnetic material which includes parallel conical bumps having a semicircular section, the bumps having N- and S-poles alternately.

4. A mattress for magnetic treatment as claimed in claim 1, wherein said permanent magnets take the form of multipolar magnets (11), each having a magnetic binding plate (12) on which magnets are arranged at all the corners, the adjacent magnets having opposite poles.

5. A mattress for magnetic treatment as claimed in claim 1, wherein there is provided a thin bag-like layer for enclosing the permanent magnets and the fiber layer.

## Patentansprüche

1. Matratze zur magnetischen Behandlung mit einem elastischen Körper (1) aus vernetztem, geschäumten Polyethylen oder geschäumtem Urethan, wobei der elastische Körper auf seiner Oberfläche Höcker zur Erzeugung chiropraktischer Effekte aufweist, mit einem an der Oberfläche des elastischen Körpers (1) befestigten Dämpfungsfilz (4), auf dem Permanentmagneten (6) punktweise verteilt sind, mit einer Faserlage (7), die an der Oberfläche des Dämpfungsfilzes (4) befestigt und zum Halten der Permanentmagneten (6) und der Höcker des elastischen Körpers (1) verwendet wird, mit einer dünner, weichen Faserlage (10), die die Oberfläche der Faserlage (7) abdeckt, wobei der elastische Körper (1) kurze (1a) und lange (1b) tonnenförmige Körper umfaßt, die miteinander verbunden und in Reihen und Spalten angeordnet sind.

2. Matratze zur magnetischen Behandlung nach Anspruch 1, wobei eine vertikale Durchgangsöffnung (5) in jedem Verbindungsabschnitt von Tonne zu Tonne vorgesehen ist.

3. Matratze zur magnetischen Behandlung nach Anspruch 1, wobei die Permanentmagneten die Polform eines magnetischen Materials annehmen, das parallele konische Höcker mit einem halbkreisförmigen Querschnitt umfaßt, wobei die Höcker abwechselnd N- und S-Pole aufweisen.

4. Matratze zur magnetischen Behandlung nach Anspruch 1, wobei die Permanentmagneten in Form von Multipol-Magneten (11) vorliegen, von denen jeder eine Magnetkopplungsplatte (12) aufweist, auf der Magnete an allen Ecken angeordnet sind, wobei benachbarte Magnete entgegengesetzte Pole aufweisen.

5. Matratze zur magnetischen Behandlung nach Anspruch 1, wobei eine dünne, taschenförmige Lage zum Umschließen der Permanentmagneten und der Faserlage vorgesehen ist.

## Revendications

1. Matelas pour traitement magnétique comprenant un corps élastique (1) de polyéthylène en mousse ou d'uréthane en mousse réticulé, le corps élastique (1) ayant des protubérances sur sa surface pour produire des effets chiropractiques; un feutre amortisseur (4) fixé à la surface du corps élastique (1), le feutre amortisseur (4) étant parsemé d'aimants permanents (6); une couche en fibre (7) liée de façon fixe à la surface du feutre amortisseur (4) et utilisée pour maintenir les aimants permanents (6) et les protubérances du corps élastique (1); une couche fine et douce en fibre (10) couvrant la surface de la couche en fibre (7); ledit corps élastique (1) comprenant des corps en forme de cylindre court (1a) et haut (1b) reliés et arrangés en lignes et colonnes.

2. Matelas pour traitement magnétique selon la revendication 1, dans lequel un trou traversant vertical (5) est prévu dans chaque portion de couplage cylindre - cylindre.

3. Matelas pour traitement magnétique selon la revendication 1, dans lequel lesdits aimants permanents prennent la forme des pôles d'un matériau magnétique qui comprend des bosses coniques parallèles possédant une section semi-circulaire, les bosses ayant des pôles -N et -S en alternance.

4. Matelas pour traitement magnétique selon la revendication 1, dans lequel lesdits aimants permanents prennent la forme d'aimants multipolaires (11), chacun ayant une plaque de liaison magnétique (12) sur laquelle les aimants sont disposés à tous les coins, les aimants adjacents possédant des pôles opposés.

5. Matelas pour traitement magnétique selon la revendication 1, dans lequel il est prévu une couche fine en forme de sac pour contenir les aimants permanents et la couche en fibre.
